# EUROPÄISCHE PATENTANMELDUNG

(11) **EP 4 647 797 A1**
(43) Veröffentlichungstag der Anmeldung: **12.11.2025**
(21) Anmeldenummer: 24175237.7
(22) Anmeldetag: 10.05.2024
(51) Int. Cl.: G01R 33/56, A61B 5/055, G06N 3/045

(54) **VERFAHREN ZUR RECHNERGESTÜTZTEN VERARBEITUNG DIGITALER BILDDATEN EINER MAGNETRESONANZEINRICHTUNG**

(71) Anmelder: Friedrich-Alexander-Universität Erlangen-Nürnberg, 91054 Erlangen (DE)
(72) Erfinder: SCHLERETH, Maja, 91058 Erlangen (DE); FAGNI, Filipp, 90408 Nürnberg (DE); DAVID, Simon, 10405 Berlin (DE); KLEYER, Arnd, 91052 Erlangen (DE); BREININGER, Katharina, 91052 Erlangen (DE)
(74) Vertreter: Fink Numrich Patentanwälte PartmbB

(57) **Zusammenfassung**

Die Erfindung beschreibt ein Verfahren zur rechnergestützten Verarbeitung digitaler Bilddaten einer Magnetresonanzeinrichtung. Das erfindungsgemäße Verfahren ist dazu konfiguriert, Messdaten eines Untersuchungsobjekts zu erfassen und die Messdaten als verschiedene MRT-Sequenzen (T1, T2, KM), welche jeweils eine Anzahl an Schnittbildern des Untersuchungsobjekts mit unterschiedlichem Bildkontrast umfassen, bereitzustellen. In dem erfindungsgemäßen Verfahren werden in einem Schritt a) Messdaten eines Untersuchungsobjekts, die verschiedene MRT-Sequenzen (T1, T2, KM) umfassen, eingelesen. In einem Schritt b) erfolgt das Ermitteln eines ausgewählten Klassifikationswerts (AKW) aus einer Mehrzahl an vorgegebenen Klassifikationswerten (KWi, i=1..N), wobei die verschiedenen MRT-Sequenzen (T1, T2, KM) einem trainierten datengetriebenen Modell (MO) mit mehreren, den verschiedenen MRT-Sequenzen (T1, T2, KM) zugeordneten, Modulen (Mx.y) als digitale Eingangsinformation zur Verfügung gestellt werden und wobei jedes der Module (Mx.y) des trainierten datengetriebenen Modells (MO) für jeden der Mehrzahl an vorgegebenen Klassifikationswerten (KWi) einen Wahrscheinlichkeitswert (WWi) als digitale Ausgangsinformation bereitstellt. Der ausgewählte Klassifikationswert (AKW) ist derjenige Klassifikationswert der Mehrzahl an vorgegebenen Klassifikationswerten (KWi), der nach einer vorgegebenen Kombination von einander aufgrund des gleichen Klassifikationswerts (KWi) zugeordneten digitalen Ausgangsinformationen jedes der Module (Mx.y) den höchsten Wahrscheinlichkeitswert (HWW) aufweist.

## Beschreibung

Die Erfindung betrifft ein Verfahren zur rechnergestützten Verarbeitung digitaler Bilddaten einer Magnetresonanzeinrichtung, die dazu konfiguriert ist, Messdaten eines Untersuchungsobjekts zu erfassen und die Messdaten als verschiedene MRT-Sequenzen, welche jeweils eine Anzahl an Schnittbildern des Untersuchungsobjekts mit unterschiedlichem Bildkontrast umfassen, bereitzustellen. Daneben betrifft die Erfindung eine Vorrichtung zur Durchführung des erfindungsgemäßen Verfahrens sowie ein Computerprogrammprodukt.

Die Magnetresonanz-Tomografie ist ein bildgebendes Verfahren zur Darstellung von Strukturen im Inneren des Körpers. Sie kann Schnittbilder des menschlichen Körpers in beliebigen Ebenen erzeugen. Aus den Daten können per Computer 3D-Datensätze berechnet werden. Die Magnetresonanz-Tomografie, MRT, nutzt die Kombination eines Magnetfelds und eines HF-Impulses zur Anregung von Wasserstoffprotonen. Befinden sich die Wasserstoffprotonen in einem starken Magnetfeld, so richten sich deren Kernspin-Achsen an den Feldlinien des Magnetfelds aus (Längsmagnetisierung). Dabei präzedieren sie wie ein Kreisel immer dichter an diese Linien heran, werden aber niemals ganz ausgerichtet.

Um für die MRT das benötigte Signal zu erhalten, wird ein kurzer Impuls einer charakteristischen Radiofrequenz über eine Antenne in das Magnetfeld eingestrahlt, wobei diese Frequenz als Larmorfrequenz bezeichnet wird. Durch den Hochfrequenzimpuls (HF-Impuls) werden die Protonen synchronisiert, wobei einige um 180° gekippt werden. Der resultierende Vektor kippt dabei in einem Koordinatensystem, in dem die X-Achse in waagerechter Richtung, die Y-Achse in senkrechter Richtung und die Z-Achse in Tiefenrichtung (Richtung der Zentralachse einer das Magnetfeld erzeugenden Magnetspule) verläuft, um 90° und dreht sich in der XY-Ebene. Anfangs kreisen die Protonen noch phasengleich, laufen aber durch verschiedene Energieabgaben an das umliegende Gewebe verschieden schnell auseinander (sog. Dephasierung) und richten sich wieder dem Magnetfeld (Z-Achse) aus.

Die sog. T1-Relaxation ist die longitudinale Relaxation und beschreibt das Zurücckippen des Vektors nach dem HF-Impuls zum Magnetfeld. Umso mehr dieser Vektor relaxiert ist, umso stärker kann er erneut angeregt werden. Bei der T1-Relaxation wird Energie an die Umgebung abgegeben. Die Zeitkonstante T1 gibt die Zeit an, in der ca. 63 % der ursprünglichen Longitudinalmagnetisierung wieder erreicht ist.

Die T2-Relaxation ist die transversale Relaxation und beschreibt den Verlust der Phasengleichheit, wo die Protonen anfangs noch phasengleich kreisen und durch die Inhomogenitäten des Magnetfeldes im Gewebe langsam dephasieren. Die Zeitkonstante T2 gibt an, wann die Transversalmagnetisierung auf 37 % des ursprünglichen Wertes zurückgegangen ist. Sie ist somit ein Maß für die Dauer des Signals. Die T1- und T2-Relaxationen sind unabhängig voneinander und laufen gleichzeitig ab.

T1 und T2 sind entscheidend für den Bildkontrast. Bei einer T1-Gewichtung gibt es andere Bilder als bei einer T2-Gewichtung. Daneben gibt es noch kontrastverstärkte Sequenzen.

Das MR-Signal entsteht durch die kreisenden Vektoren in der XY-Ebene, angeregt durch die eingestreute Larmorfrequenz. Das Gesamtsignal (MR-Signal) kann von einer Antenne des MRT empfangen werden. Das MR-Signal ist abhängig von der Protonendichte, vom Magnetfeld B₀ des Tomographen, von der T1-Relaxationszeit (wie schnell das Gewebe wieder erregt werden kann) und der T2-Relaxationszeit (Länge des Signals). Die Protonendichte sowie T1 und T2 sind dabei vom Gewebe abhängig, woraus sich die verschiedenen Kontraste berechnen lassen. Dies ist die Grundlage der Magnetresonanzbildgebung.

Um ein MR-Bild zu erhalten, muss eine Schicht mehrmals angeregt und gemessen werden. Der Bildkontrast wird dabei durch die T1-Gewichtung (Repetitionszeit) und die T2-Gewichtung (Echozeit) bestimmt. Diese Repetitionszeit ist die Zeit zwischen den Anregungen, in der sich die Protonen wieder dem Magnetfeld ausrichten können. Je länger diese Zeit ist, umso größer ist die Längsmagnetisierung der Protonen und umso stärker wird das Signal bei erneuter Anregung. Die Echozeit ist die Zeit zwischen der Anregung und der Messung des MR-Signals.

Die MRT wird beispielsweise dazu verwendet, den Schweregrad von Krankheitsmustern, wie z.B. chronisch-entzündliche rheumatische Gelenkerkrankungen (wie rheumatoide Arthritis, RA) und Spondyloarthritiden (wie Psoriasis-Arthritis, PsA) zu bewerten. Diese sind progressive immunvermittelte Krankheiten, die mit umfangreicher Gelenkdestruktion, Funktionsverlust und somit mit zunehmender irreversibler Behinderung einhergehen. Vorrangig sind die Gelenke in der Hand betroffen. Die ersten und häufigsten Krankheitsmuster sind entzündliche Läsionen wie Synovitis, Tenosynovitis und Osteitis. Obwohl diese Muster, die auch als Pathologien bezeichnet werden, teilweise durch immunsupprimierende Medikation umkehrbar sind, tragen sie hauptsächlich zur Krankheitsaktivität bei und sind direkt mit einem erhöhten Risiko für erosive Knochenläsionen verknüpft.

Knochenerosionen sind irreversible strukturelle Veränderungen und treten in schweren unkontrollierten Gelenksentzündungen auf. Die Neuentwicklung von Erosionen in RA und PsA ist ein prognostischer Hauptfaktor für die Krankheitsentwicklung und reduziertes Therapieansprechen und ist assoziiert mit erhöhter Mortalität. Die Reduktion von neuen Handerosionen wird in bildgebenden Verfahren aufgrund der prognostischen Relevanz häufig als primärer Endpunkt in RA- und PsA-Studien verwendet, um die Auswirkungen von Medikamenten und anderen therapeutischen Interventionen zu untersuchen.

Üblicherweise werden zur Bewertung des Schweregrades verschiedene MRT-Sequenzen der Hand manuell ausgewertet. Eine jeweilige MRT-Sequenz umfasst eine Anzahl an Schnittbildern des Untersuchungsobjekts, d.h. der Hand bzw. einzelner Gelenke der Hand, mit unterschiedlichem Bildkontrast. Dabei werden bei RA und PsA verschiedene relevante Regionen, sog. ROIs, untersucht und mit einem Wert zwischen 0 und 10 für verschiedene Krankheitsmuster bewertet und klassifiziert. Die wichtigsten Krankheitsmuster sind hierbei Synovitis, Osteitis und Erosionen. Die einzelnen Werte der jeweiligen ROIs werden anschließend aufsummiert. Dann wird ein gesamter, international validierter Krankheitsscore bemessen [1, 2]. Diese manuelle Auswertung ist sehr zeitaufwendig und benötigt einen erfahrenen Rheumatologen oder Radiologen. Zudem besteht das Problem, dass es trotz hoher Übereinstimmung zwischen verschiedenen Experten mit einem Interklassenkorrelationskoeffizienten (ICC) zwischen 0.74 und 0.95 einen Bedarf gibt, das Bewertungsverfahren objektiver zu gestalten und insbesondere zu beschleunigen.

Es ist Aufgabe der Erfindung, ein Verfahren anzugeben, mit dem eine rechnergestützte Verarbeitung digitaler Bilddaten einer Magnetresonanzeinrichtung auf einfache und zuverlässige Weise erfolgen kann. Eine weitere Aufgabe der Erfindung besteht darin, eine Vorrichtung anzugeben, die dazu eingerichtet ist, ein erfindungsgemäßes Verfahren auszuführen. Eine weitere Aufgabe der Erfindung besteht darin, ein Computerprogrammprodukt anzugeben.

Diese Aufgaben werden gelöst durch ein Verfahren gemäß den Merkmalen des Anspruches 1, ein Computerprogrammprodukt gemäß den Merkmalen des Anspruches 12 und eine Vorrichtung gemäß den Merkmalen des Anspruches 13. Vorteilhafte Ausgestaltungen ergeben sich aus den abhängigen Ansprüchen.

Gemäß einem ersten Aspekt der vorliegenden Erfindung wird ein Verfahren zur rechnergestützten Verarbeitung digitaler Bilddaten einer Magnetresonanzeinrichtung vorgeschlagen, die dazu konfiguriert ist, Messdaten eines Untersuchungsobjekts zu erfassen, und die Messdaten als verschiedene MRT-Sequenzen bereitzustellen. Eine jeweilige MRT-Sequenz umfasst jeweils eine Anzahl an Schnittbildern des Untersuchungsobjekts mit unterschiedlichem Bildkontrast.

Insbesondere können die verschiedenen MRT-Sequenzen eine koronare T1-gewichtete MRT-Sequenz, eine T2-gewichtete MRT-Sequenz und eine, insbesondere fettunterdrückte, kontrastverstärkte MRT-Sequenz umfassen. Ebenso können beispielsweise Protonendichte-gewichtete und diffusionsgewichtete Aufnahmen für die verschiedenen MRT-Sequenzen verwendet werden. Auch andere MRT-Sequenzen sind möglich. Es ist jedoch zweckmäßig, wenn MRT-Sequenzen verschiedener Kontraste verwendet werden.

Als Untersuchungsobjekt wird insbesondere eine Hand eines Menschen betrachtet. Genauer werden hierbei die einzelnen Gelenke der verschiedenen Finger betrachtet. Das vorliegende Verfahren kann dabei für jedes einzelne Gelenk separat zur Anwendung kommen. Es sind auch andere Untersuchungsobjekte denkbar.

Bei dem erfindungsgemäßen Verfahren werden die folgenden Schritte durchgeführt:
In einem Schritt a) erfolgt ein Einlesen von Messdaten des Untersuchungsobjekts, die verschiedene MRT-Sequenzen umfassen.

In einem Schritt b) erfolgt ein Ermitteln eines ausgewählten Klassifikationswerts aus einer Mehrzahl an vorgegebenen Klassifikationswerten, wobei die verschiedenen MRT-Sequenzen einem trainierten datengetriebenen Modell mit mehreren, den verschiedenen MRT-Sequenzen zugeordneten, Modulen als digitale Eingangsinformation zur Verfügung gestellt werden. Jedes der Module des trainierten datengetriebenen Modells stellt für jeden der Mehrzahl an vorgegebenen Klassifikationswerten einen Wahrscheinlichkeitswert als digitale Ausgangsinformation bereit. Der ausgewählte Klassifikationswert ist derjenige Klassifikationswert der Mehrzahl an vorgegebenen Klassifikationswerten, der nach einer vorgegebenen Kombination voneinander aufgrund des gleichen Klassifikationswerts zugeordneten digitalen Ausgangsinformationen jedes der Module den höchsten Wahrscheinlichkeitswert aufweist.

Bei den Klassifikationswerten kann es sich z.B. um Kategorien, ordinal skalierte Werte oder kardinal skalierte Werte handeln.

Mit dem erfindungsgemäßen Verfahren ist es erstmals möglich, automatisiert und rechnergestützt digitale Bilddaten einer Magnetresonanzeinrichtung zu verarbeiten und dabei die für eine Klassifikation der Krankheitsschwere eines Untersuchungsobjekts erforderlichen Klassifikationswerte automatisiert zu bestimmen. Ermöglicht wird dies durch das Bereitstellen jeweiliger Module in einem trainierten datengetriebenen Modell, welche basierend auf den verschiedenen MRT-Sequenzen einen Klassifikationswert bestimmen, die miteinander kombiniert die Bestimmung des Klassifikationswerts ermöglichen, der die höchste Wahrscheinlichkeit der vorgegebenen Klassifikationswerte aufweist.

Durch die automatische Bewertung ist eine vereinfachte, schnellere, quantitativere und Untersucher-unabhängige Bewertung verschiedener Krankheitsbilder eines Untersuchungsobjekts, insbesondere einer Hand von Patienten mit rheumatischer Arthritis RA und psoriatischer Arthritis PsA möglich. Dies ermöglicht eine vereinfachte Verlaufskontrolle mit einem konstanten quantitativen Bewertungsverfahren.

Das Verfahren ermöglicht die automatische Bewertung unterschiedlicher Pathologien, insbesondere von Erosionen, Osteitis und Synovitis, bei Patienten mit RA und PsA mittels eines datengetriebenen Modells unter Nutzung von MRT-Aufnahmen.

Gemäß einer zweckmäßigen Ausgestaltung ist vorgesehen, dass die Module des datengetriebenen Modells auf einer Mehrzahl von Maschinenlernverfahren, insbesondere neuronalen Netzwerken wie Deep Neural Networks, basieren.

Eine weitere zweckmäßige Ausgestaltung sieht vor, dass die Module des datengetriebenen Modells für jede der verschiedenen MRT-Sequenzen auf einem jeweiligen, trainierten neuronalen Netzwerk basieren.

Es ist weiterhin vorgesehen, dass für unterschiedliche Pathologien des Untersuchungsobjekts jeweilige Module des datengetriebenen Modells vorgesehen werden, die jeweils auf einem unterschiedlichen, trainierten neuronalen Netzwerk basieren. Wie bereits beschrieben, werden als Pathologien insbesondere Erosionen, Osteitis und Synovitis berücksichtigt.

Es ist weiter zweckmäßig, wenn die für eine bestimmte Pathologie verwendeten Module verschiedener MRT-Sequenzen miteinander verknüpft sind, wobei die Verknüpfung durch das Trainieren der verknüpften Module angelegt ist. Eine solche Verknüpfung kann beispielsweise Vorwissen oder Wissen aus Studien repräsentieren.

Es ist weiterhin zweckmäßig, wenn jedes der Module des datengetriebenen Modells für jeden der Mehrzahl an vorgegebenen Klassifikationswerten einen Wahrscheinlichkeitswert bestimmt. Mit anderen Worten ist jedes der Module des datengetriebenen Modells entsprechend trainiert, für die Mehrzahl an vorgegebenen Klassifikationswerten einen Wahrscheinlichkeitswert zu ermitteln.

Eine weitere zweckmäßige Ausgestaltung sieht vor, dass eine Verknüpfung der für jeden der Mehrzahl an vorgegebenen Klassifikationswerten bestimmten Wahrscheinlichkeitswerte erfolgt, wobei eine Verknüpfung solcher Wahrscheinlichkeitswerte erfolgt, welche für einen entsprechenden Klassifikationswert ermittelt wurden.

Die Verknüpfung kann insbesondere folgendes umfassen:
- eine Mittelwertbildung der Wahrscheinlichkeitswerte entsprechender Klassifikationswerte;
- eine Gewichtung der Wahrscheinlichkeitswerte der unterschiedlichen MRT-Sequenzen mit unterschiedlichen Faktoren, wobei die Faktoren z.B. aus Studien abgeleitet sein können oder auf Vorwissen basieren;
- eine Auswahl des Klassifikationswerts, der den höchsten Wahrscheinlichkeitswert aller entsprechenden Klassifikationswerte aufweist;
- eine Auswahl des Klassifikationswerts, welcher ein höchstes Vertrauensmaß hat.

In einer besonders bevorzugten Ausgestaltung ist die Anzahl an vorgegebenen Klassifikationswerten vier oder mehr. Die Klassifikation kann auch derart vorgesehen sein, dass ein Klassifikationswert eine Mehrzahl von Klassifikationswerten umfasst.

Die Anzahl an vorgegebenen Klassifikationswerten ist aus einem der folgenden Bewertungsverfahren abgeleitet: RAMRIS ([3]) oder PsAMRIS ([4]). Dies sind international gängige Bewertungsmethodiken für MRT Bildgebung der Hand bei rheumatoider Arthritis und psoriatischer Arthritis.

Es ist weiterhin zweckmäßig, wenn jedes der verschiedenen Module für jede der verschiedenen MRT-Sequenzen gesondert trainiert ist.

Gemäß einem zweiten Aspekt wird eine Vorrichtung zur rechnergestützten Verarbeitung digitaler Bilddaten einer Magnetresonanzeinrichtung vorgeschlagen, die dazu konfiguriert ist, Messdaten eines Untersuchungsobjekts, insbesondere einer Hand, zu erfassen und als verschiedene MRT-Sequenzen bereitzustellen. Die Vorrichtung umfasst einen Prozessor, der dazu eingerichtet ist, die Schritte des Verfahrens gemäß einer oder mehrerer Ausgestaltungsvarianten durchzuführen.

Weiter umfasst die Erfindung ein Computerprogrammprodukt mit Programmcode, der auf einem nicht-flüchtigen maschinenlesbaren Träger gespeichert ist zur Durchführung eines Verfahrens gemäß einer oder mehrerer Ausgestaltungsvarianten, wenn der Programmcode auf einem Computer ausgeführt wird.

Die Erfindung wird nachfolgend näher anhand eines Ausführungsbeispiels in der Zeichnung erläutert. Es zeigen:
- Fig. 1: eine schematische Darstellung gemäß einer ersten Ausführungsform, welche die Schritte des erfindungsgemäßen Verfahrens illustriert; und
- Fig. 2: eine schematische Darstellung gemäß einer zweiten Ausführungsform, welche die Schritte des erfindungsgemäßen Verfahrens illustriert.

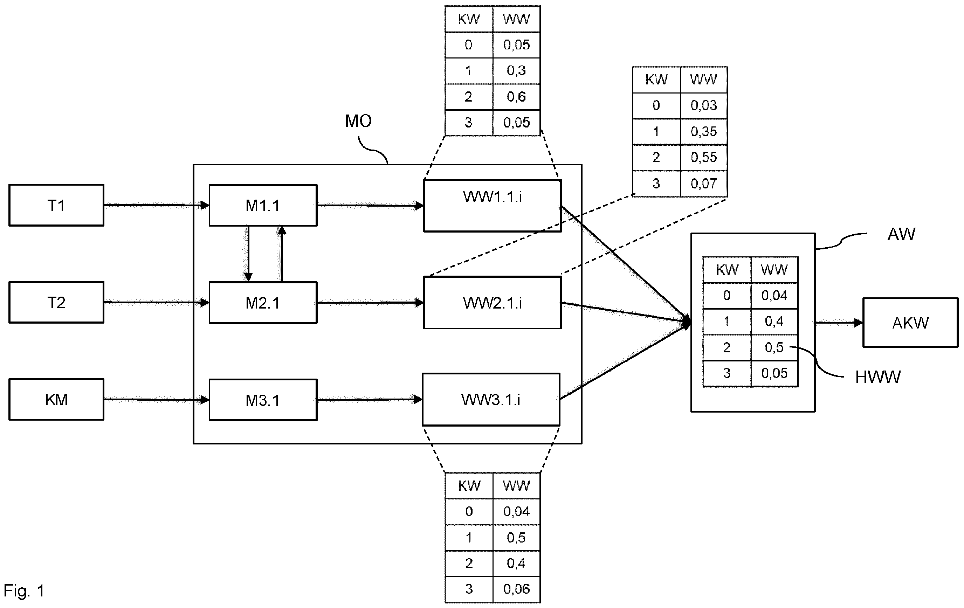

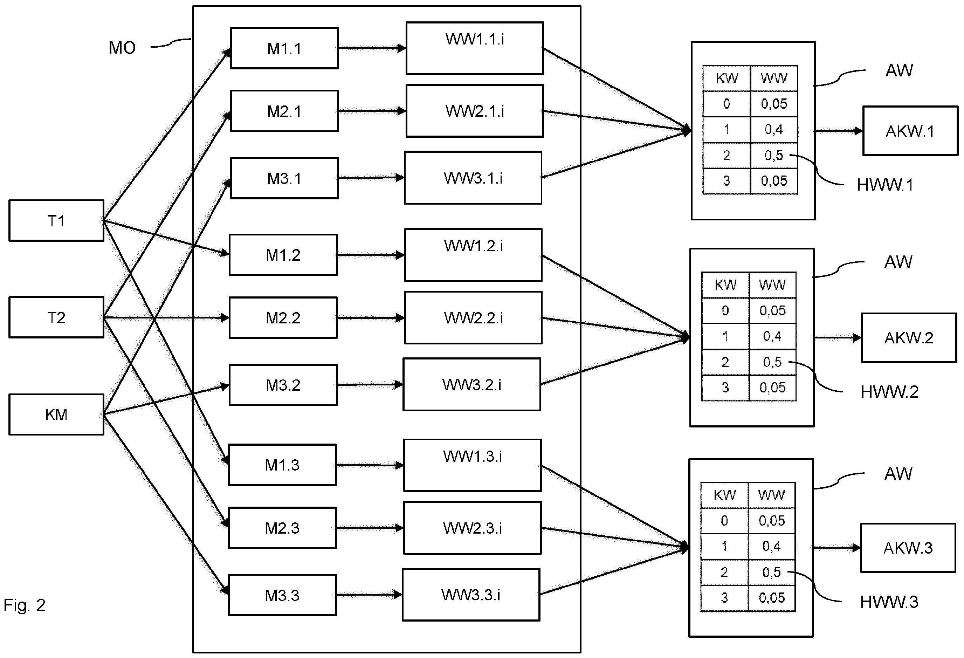

Das vorliegende Verfahren zur rechnergestützten Verarbeitung digitaler Bilddaten nutzt Bilddaten, die von einer in den Figuren nicht dargestellten Magnetresonanzeinrichtung bereitgestellt werden. Eine solche, an sich bekannte Magnetresonanzeinrichtung ist dazu konfiguriert, Messdaten eines ebenfalls in den Figuren nicht dargestellten Untersuchungsobjekts zu erfassen und die Messdaten als verschiedene MRT-Sequenzen bereitzustellen. Eine jeweilige MRT-Sequenz umfasst in bekannter Weise eine Anzahl an Schnittbildern des Untersuchungsobjekts, wobei die Bilder unterschiedlicher MRT-Sequenzen unterschiedlichen Bildkontrast aufweisen. Bevorzugt werden im Rahmen des erfindungsgemäßen Verfahrens als MRT-Sequenzen eine koronare T1-gewichtete MRT-Sequenz (in den Figuren 1 und 2 nachfolgend: T1), eine T2-gewichtete MRT-Sequenz (nachfolgend: T2) und eine, insbesondere fettunterdrückte, kontrastverstärkte MRT-Sequenz (nachfolgend: KM) verarbeitet.

Als Untersuchungsobjekt wird insbesondere eine Hand eines Menschen betrachtet. Das nachfolgend beschriebene Vorgehen wird hierbei vorzugsweise iterativ für die einzelnen Gelenke der verschiedenen Finger durchgeführt. Alternativ kann das Verfahren auch für andere Gelenke eines Menschen oder Tiers durchgeführt werden.

Die Bilder der verschiedenen MRT-Sequenzen werden dazu verwendet, den Schweregrad von Krankheitsmustern, insbesondere rheumatoider Arthritis (RA) und Spondyloarthritiden (PsA) zu bewerten. Als Krankheitsmuster (auch als Pathologien bezeichnet) werden mit Hilfe des erfindungsgemäßen Verfahrens entzündliche Läsionen wie Synovitis und Osteitis sowie Knochenerosionen (kurz: Erosionen) klassifiziert, wobei die genannten Läsionen nicht als abschließend, sondern lediglich beispielhaft zu betrachten sind.

Die Erzeugung der Messdaten als verschiedene MRT-Sequenzen T1, T2, KM, die jeweils eine Anzahl an Schnittbildern des Untersuchungsobjekts mit unterschiedlichem Bildkontrast umfassen, erfolgt in einer dem Fachmann bekannten Weise und wird daher nicht näher im Detail beschrieben.

Bei dem erfindungsgemäßen Verfahren werden, wie in den Figuren 1 und 2 dargestellt, in einem ersten Schritt die Messdaten eines Untersuchungsobjekts, z.B. eines Gelenks der Hand, als die verschiedenen MRT-Sequenzen T1, T2 und KM eingelesen. Die verschiedenen MRT-Sequenzen T1, T2 und KM werden als Eingangsinformationen einem trainierten datengetriebenen Modell MO zur Verfügung gestellt.

Das datengetriebene Modell MO umfasst im ersten Ausführungsbeispiel gemäß Fig. 1 drei Module M1.1, M2.1 und M3.1. Jedes der Module M1.1 bis M3.1 basiert auf einer Mehrzahl von unabhängig voneinander trainierten neuronalen Netzwerken oder anderen Methoden des maschinellen Lernens. Bei den neuronalen Netzwerken handelt es sich insbesondere um Deep Neural Networks. Das Suffix ".1" der Module M1.1 bis M3.1 repräsentiert ein erstes betrachtetes Krankheitsmuster y, z.B. eine Erosion. Der Term "M1", "M2" und "M3" repräsentiert eine jeweilige MRT-Sequenz T1 oder T2 oder KM.

Die neuronalen Netzwerke der Module M1.1 und M2.1 sind beispielhaft miteinander verknüpft, d.h. voneinander abhängig, was durch die Pfeile zwischen den beiden Modulen M1.1 und M2.1 symbolisiert ist. Die Verknüpfung ist durch das Trainieren der miteinander verknüpften Module, hier M1.1 und M2.1, angelegt.

Jedes der Module M1.1 bis M3.1 ist dazu eingerichtet für eine Mehrzahl an vorgegebenen Klassifikationswerten KWi (i = 0 bis 3) jeweils einen Wahrscheinlichkeitswert WWi (i = 0 bis 3) als digitale Ausgangsinformation WW1.1.i, WW2.1.i und WW3.1.i bereitzustellen. In Fig. 1 ist dies durch die den Ausgangsinformationen WW1.1.i, WW2.1.i und WW3.1.i angehängten Tabellen symbolisiert. Die Klassifikationswerte KWi entsprechen insbesondere einem Intraklassenkorrelationskoeffizienten ICC, der auch als Score bezeichnet wird. Ein Klassifikationswert 0 in der genannten Klassifikationsmetrik entspricht "keiner Aktivität", während der Klassifikationswert 3 einer "hohen Aktivität" entspricht, welche jeweils die Krankheitsaktivität bemessen. Die Klassifikationswerte sind z.B. aus dem RAMRIS bzw. PsAMRIS-Bewertungsverfahren abgeleitet, welches Klassifikationswerte (Scores) zwischen 0 und 10 vergibt. In dem hier gewählten Ausführungsbeispiel wurden Klassifikationswerte höher als 2 in Klasse 3 eingruppiert. Dieses Vorgehen kann jedoch in geeigneter Weise angepasst werden.

Die den Klassifikationswerten KWi zugeordneten bzw. für diese ermittelten Wahrscheinlichkeitswerte WWi werden in einem weiteren Schritt AW in vorgegebener Weise miteinander verknüpft bzw. kombiniert, wobei die Kombination voneinander aufgrund des gleichen Klassifikationswerts zugeordneten digitalen Ausgangsinformationen jedes der Module M1.1-M3.1 erfolgt. Dies ist in der Tabelle im Schritt AW dargestellt. Es wird schließlich derjenige Klassifikationswert AKW ausgewählt, der in der Tabelle im Schritt AW den höchsten Wahrscheinlichkeitswert HWW aufweist. Im vorliegenden Ausführungsbeispiel der Fig. 1 ist dies der Klassifikationswert 2, da dieser mit dem Wahrscheinlichkeitswert 0,5 den höchsten Wahrscheinlichkeitswert HWW aller Wahrscheinlichkeitswerte 0,05 (für den Klassifikationswert 0), 0,4 (für den Klassifikationswert 1), 0,5 (für den Klassifikationswert 2) und 0,05 (für den Klassifikationswert 3) aufweist. Dieser so ermittelte Klassifikationswert wird für das abschließende Scoring, das gemäß dem Stand der Technik erfolgt und hier nicht näher beschrieben ist, verwertet.

Die Bestimmung der Wahrscheinlichkeitswerte WWi im Schritt AW aus den Klassifikationswerten KWi zugeordneten Wahrscheinlichkeitswerten WWi der Ausgangsinformationen WWx.y.i (mit x = 1, 2, 3 für die MRT-Sequenzen T1, T2, KM und y = 1 für die betrachtete Läsion) erfolgt anhand einer Verknüpfung, wobei die Verknüpfung solcher Wahrscheinlichkeitswerte erfolgt, welche dem entsprechenden Klassifikationswert KWi zugeordnet sind. Dies bedeutet, es werden diejenigen Wahrscheinlichkeitswerte WWi der Ausgangsinformationen WW1.1.i, WW2.1.i und WW3.1.i miteinander verknüpft, die z.B. dem Klassifikationswert 0 usw., zugeordnet sind.

Die Verknüpfung kann beispielsweise eine Mittelwertbildung der Wahrscheinlichkeitswerte WWi entsprechender Klassifikationswerte KWi umfassen. In dem in Fig. 1 gezeigten Ausführungsbeispiel kann beispielsweise der Mittelwert aus den Wahrscheinlichkeitswerten für den Klassifikationswert 0 gebildet werden gemäß (0,05 + 0,03 + 0,04)/3 = 0,04, was dem Wahrscheinlichkeitswert für den Klassifikationswert 0 im Schritt AW entspricht. Für die Klassifikationswerte KW1, KW2, KW3 wird eine entsprechende Verknüpfung durch Mittelwertbildung vorgenommen.

Alternativ kann eine Gewichtung der Wahrscheinlichkeitswerte WWi der unterschiedlichen MRT-Sequenzen T1, T2, KM mit vorgegebenen Faktoren erfolgen. Solche Faktoren können beispielsweise aus Studien abgeleitet sein oder aus entsprechendem Vorwissen erzeugt sein. In diesem Fall würde sich für den Klassifikationswert KW1 in Schritt AW die Berechnung wie folgt ergeben: a × 0,3 + b × 0,35 + c × 0,5 = 0,4. Die Parameter a, b und c bilden dann die genannten Faktoren, die vorbekannt sind.

Alternativ kann die Verknüpfung eine Auswahl des Klassifikationswerts KWi umfassen, der den höchsten Wahrscheinlichkeitswert aller entsprechenden Klassifikationswerte aufweist. Für den in Figur 1 gezeigten Klassifikationswert KW2 (d.h. i = 2) mit den Wahrscheinlichkeitswerten WW1.1.2 = 0,6, WW2.1.2 = 0,55 und WW3.1.2 = 0,4 würde sich, da der Wahrscheinlichkeitswert der MRT-Sequenz T1 am höchsten ist, 0,6 ergeben.

In einer weiteren alternativen Ausgestaltung kann die Verknüpfung eine Auswahl des Klassifikationswerts KWi umfassen, welcher ein höchstes Vertrauensmaß hat. Ein solches des Vertrauensmaß kann beispielsweise für die Wahrscheinlichkeitswerte WWi für die MRT-Sequenz T1 vorliegen, bei dem hier beispielhaft die geringste Unsicherheit vorliegt. Zur Bestimmung der Unsicherheit bzw. des Vertrauensmaßes können hierbei geeignete Verfahren wie beispielsweise Bayes`sche Ansätze genutzt werden. Auch in diesem Fall würde der Klassifikationswert KW2 ausgewählt werden.

Das in Fig. 1 gezeigte Ausführungsbeispiel basiert beispielhaft auf insgesamt vier Klassifikationswerten KW0, KW1, KW2, KW3. Wie erläutert, kann die Anzahl an Klassifikationswerten bzw. Scores auch größer als 3 gewählt werden. Im vorliegenden Ausführungsbeispiel wurden die Module des trainierten datengetriebenen Netzes derart trainiert, dass in der Klasse für den Klassifikationswert 3 alle Scores einfließen, die größer als 2 sind.

Fig. 2 zeigt ein weiteres Ausführungsbeispiel, bei dem das datengetriebene trainierte Modell MO eine größere Anzahl an Modulen M1.1 bis M3.3 umfasst, wobei die Module dem Suffix ".2" und ".3" zur Erkennung anderer Krankheitsmuster, z.B. Osteitis oder Synovitis, trainiert sind. Im Gegensatz zum vorangegangenen Ausführungsbeispiel gemäß Fig. 1 sind in diesem Ausführungsbeispiel auch keine Verknüpfungen zwischen einzelnen Modulen des trainierten datengetriebenen Modells MO vorgesehen. Es versteht sich, dass solche Verknüpfungen innerhalb der Module für ein Krankheitsmuster oder mehrere Krankheitsmuster vorhanden sein können.

Die weitere Bestimmung des ausgewählten Klassifikationswertes aus einer Mehrzahl an vorgegebenen Klassifikationswerten KWi erfolgt in der oben beschriebenen Weise, wobei für jedes Krankheitsmuster ".1", ".2", ".3" und jede MRT-Sequenz T1, T2, KM ein jeweiliges Modul in dem datengetriebenen Modell MO vorgesehen ist. Jedes der Module Mx.y des datengetriebenen Modells MO stellt für jeden der Mehrzahl an vorgegebenen Klassifikationswerten KWi, welche für jedes Modul gleich sind, einen Wahrscheinlichkeitswert WWi als digitale Ausgangsinformation bereit. Der ausgewählte jeweilige Klassifikationswert AKW.1, AKW.2, AKW.3 (allgemein: AKW.y) ist dann derjenige Klassifikationswert der Mehrzahl an vorgegebenen Klassifikationswerten KWi, der nach einer vorgegebenen Kombination voneinander aufgrund des gleichen Klassifikationswerts KWi zugeordneten digitalen Ausgangsinformationen jedes der Module Mx.1 bzw. Mx.2 bzw. Mx.3 den höchsten Wahrscheinlichkeitswert HWW1. Bzw. HWW.2 bzw. HWW.3 aufweist.

In dem in Fig. 2 ausgeführten Ausführungsbeispiel ist jedes der verschiedenen Module Mx.y für jede der verschiedenen MRT-Sequenzen T1, T2, KM gesondert trainiert. Insbesondere bestimmt jedes der Module Mx.y des datengetriebenen Modells MO für jeden der Mehrzahl an vorgegebenen Klassifikationswerten KWi einen jeweiligen Wahrscheinlichkeitswert WWi.

In dem vorliegenden Verfahren werden damit mehrere neuronale Netzwerke genutzt, um automatisiert und rechnergestützt in den jeweils ausgewählten Regionen eines Untersuchungsobjekts (Gelenk) verschiedene Krankheitsmuster zu erkennen. Dazu werden als Eingaben die entsprechenden Bereiche der MRT-Sequenzen verwendet. Die jeweiligen neuronalen Netzwerke werden vollüberwacht durch manuell annotierte Bilddaten trainiert.

Durch das automatische, rechnergestützte Bewertungsverfahren ist eine vereinfachte, schnellere, quantitativere und untersuchungsunabhängige Bewertung verschiedener Krankheitsbilder, insbesondere des Untersuchungsobjekts möglich, wobei das Verfahren für die Erkennung von rheumatischer Arthritis RA und psoriatischer Arthritis PsA möglich ist. Dies ermöglicht eine vereinfachte Verlaufskontrolle mit einem konstanten, quantitativen Bewertungsverfahren. Das Verfahren ermöglicht zudem die automatische Bewertung von Erosionen, Osteitis und Synovitis bei Patienten mit RA und PsA mittels Maschinenlernverfahren in MRT-Aufnahmen.

Wie beschrieben, wird für verschiedene MRT-Sequenzen (koronale T1-gewichtete MRT-Sequenzen und T1- und T2-gewichtete fettunterdrückte kontrastverstärkte Sequenzen) jeweils ein separates Modul trainiert. Hierzu kann beispielsweise ResNet-3D verwendet werden, was auf einem Kinetics400-Datensatz vortrainiert oder nicht vortrainiert wurde. Die neuronalen Netzwerke der Module werden vorzugsweise mit mehreren hundert Patientendaten trainiert und auf separaten Kohorten validiert. Konkret werden in den einzelnen MRT-Bildern jeweils die zu untersuchenden Gelenke und Mittelhandknochen annotiert und anschließend als sog. ROIs (Regions of Interest) extrahiert. Für jede der Pathologien ist ein trainiertes neuronales Netzwerk als Modul verfügbar und bewertet diese jeweils mit einem Klassifikationswert zwischen 0 (keine Aktivität und 3+ (hohe Aktivität), der die Krankheitsaktivität bemisst. Diese Scores sind aus den bekannten Bewertungsverfahren RAMRIS bzw. PsAMRIS abgeleitet, welche Scores zwischen 0 und 10 vergeben. Scores höher 2 werden in die Klasse 3+ eingruppiert. Die Wahrscheinlichkeiten der einzelnen Klassen-Netzwerkvorhersagen werden abschließend über die jeweiligen einzelnen MRT-Sequenzen verknüpft und das Mehrheitsvotum für das abschließende Scoring verwendet.

In dem datengetriebenen Modell MO ist somit vorgesehen, jede der verschiedenen MRT-Sequenzen einzeln in einem separaten neuronalen Netzwerk (Modul) zu trainieren. Die Ausgabeinformationen der einzelnen Module werden dann miteinander verknüpft. Für Synovitis kann eine fokusbasierte Verlustfunktion verwendet werden, um kleinere Datensätze auszugleichen. Zusätzlich kann ein Gesamt-Klassifikationswert für das gesamte Untersuchungsobjekt pro Krankheitsmuster bereitgestellt werden.

### Referenzen

1 Taylor W, Gladman D, Helliwell P, et al. Classification criteria for psoriatic arthritis: development of new criteria from a large international study. Arthritis Rheum 2006;54(8):2665-73.
2 Kay J, Upchurch KS. ACR/EULAR 2010 rheumatoid arthritis classification criteria. Rheumatology (Oxford) 2012;51 Suppl 6(suppl_6):vi5-9. https://academic.oup.com/rheumatology/article/51/suppl_6/vi5/1787592.
3 Haavardsholm, E. A., Østergaard, M., Ejbjerg, B. J., Kvan, N. P., Uhlig, T. A., Lilleäs, F. G., & Kvien, T. K. (2005). Reliability and sensitivity to change of the OMERACT rheumatoid arthritis magnetic resonance imaging score in a multireader, longitudinal setting. Arthritis & Rheumatism, 52(12), 3860-3867.
4 Østergaard, M., McQUEEN, F. I. O. N. A., Wiell, C., Bird, P., Bøyesen, P., Ejbjerg, B., ... & Conaghan, P. G. (2009). The OMERACT psoriatic arthritis magnetic resonance imaging scoring system (PsAMRIS): definitions of key pathologies, suggested MRI sequences, and preliminary scoring system for PsA Hands. The Journal of rheumatology, 36(8), 1816-1824.

### Bezugszeichenliste

- T1: erste MRT-Sequenz
- T2: zweite MRT-Sequenz
- KM: dritte MRT-Sequenz
- MO: datengetriebenes Modell
- Mx.y: Modul des datengetriebenen Modells, mit x = Pathologie 1, 2, 3 und y = MRT-Sequenz
- KWi: Klassifikationswert der Klasse i, i=1...N
- WWi: Wahrscheinlichkeitswert der Klasse i
- WWx.y.i: Ausgangswahrscheinlichkeitswert der MRT-Sequenz x, der Pathologie y und der Klasse i
- AKW: ausgewählten Klassifikationswert
- HWW: höchster Wahrscheinlichkeitswert

## Patentansprüche

1. Verfahren zur rechnergestützten Verarbeitung digitaler Bilddaten einer Magnetresonanzeinrichtung, die dazu konfiguriert ist, Messdaten eines Untersuchungsobjekts zu erfassen und die Messdaten als verschiedene MRT-Sequenzen (T1, T2, KM), welche jeweils eine Anzahl an Schnittbildern des Untersuchungsobjekts mit unterschiedlichem Bildkontrast umfassen, bereitzustellen, bei dem die folgenden Schritte durchgeführt werden:
a) Einlesen von Messdaten eines Untersuchungsobjekts, die verschiedene MRT-Sequenzen (T1, T2, KM) umfassen;
b) Ermitteln eines ausgewählten Klassifikationswerts (AKW) aus einer Mehrzahl an vorgegebenen Klassifikationswerten (KWi, i=1..N), wobei die verschiedenen MRT-Sequenzen (T1, T2, KM) einem trainierten datengetriebenen Modell (MO) mit mehreren, den verschiedenen MRT-Sequenzen (T1, T2, KM) zugeordneten, Modulen (Mx.y) als digitale Eingangsinformation zur Verfügung gestellt werden und wobei jedes der Module (Mx.y) des trainierten datengetriebenen Modells (MO) für jeden der Mehrzahl an vorgegebenen Klassifikationswerten (KWi) einen Wahrscheinlichkeitswert (WWi) als digitale Ausgangsinformation bereitstellt, wobei der ausgewählte Klassifikationswert (AKW) derjenige Klassifikationswert der Mehrzahl an vorgegebenen Klassifikationswerten (KWi) ist, der nach einer vorgegebenen Kombination von einander aufgrund des gleichen Klassifikationswerts (KWi) zugeordneten digitalen Ausgangsinformationen jedes der Module (Mx.y) den höchsten Wahrscheinlichkeitswert (HWW) aufweist.

2. Verfahren nach Anspruch 1, bei dem die Module (Mx.y) des datengetriebenen Modells (MO) auf einer Mehrzahl von Maschinenlernverfahren, insbesondere neuronalen Netzwerken, basieren.

3. Verfahren nach Anspruch 1 oder 2, bei dem die Module (Mx.y) des datengetriebenen Modells (MO) für jede der verschiedenen MRT-Sequenzen (T1, T2, KM) auf einem jeweiligen, trainierten neuronalen Netzwerk basieren.

4. Verfahren nach einem der vorhergehenden Ansprüche, bei dem die verschiedenen MRT-Sequenzen eine koronare T1-gewichtete MRT-Sequenz, eine T2-gewichtete MRT-Sequenz und eine, insbesondere fett unterdrückte, kontrastverstärkte MRT-Sequenz umfasst.

5. Verfahren nach einem der vorhergehenden Ansprüche, bei dem für unterschiedliche Pathologien des Untersuchungsobj ekts jeweilige Module (Mx.y) des datengetriebenen Modells (MO) vorgesehen werden, die jeweils auf einem unterschiedlichen, trainierten neuronalen Netzwerk basieren.

6. Verfahren nach Anspruch 4 und 5, bei dem die für eine bestimmte Pathologie verwendeten Module (Mx.y) verschiedener MRT-Sequenzen (T1, T2, KM) miteinander verknüpft sind, wobei die Verknüpfung durch das Trainieren der verknüpften Module (Mx.y) angelegt ist.

7. Verfahren nach einem der vorhergehenden Ansprüche, bei dem jedes der Module (Mx.y) des datengetriebenen Modells (MO) für jeden der Mehrzahl an vorgegebenen Klassifikationswerten (KWi) einen Wahrscheinlichkeitswert (WWx.y.i) bestimmt.

8. Verfahren nach Anspruch 7, bei dem eine Verknüpfung der für jeden der Mehrzahl an vorgegebenen Klassifikationswerten (KWi) bestimmten Wahrscheinlichkeitswerte (WWx.y.i) erfolgt, wobei eine Verknüpfung solcher Wahrscheinlichkeitswerte (WWx.y.i) erfolgt, welche für einen entsprechenden Klassifikationswert (KWi) ermittelt wurden.

9. Verfahren nach Anspruch 7 oder 8, bei dem die Verknüpfung umfasst:
- eine Mittelwertbildung der Wahrscheinlichkeitswerte (WWx.y.i) entsprechender Klassifikationswerte (KWi);
- eine Gewichtung der Wahrscheinlichkeitswerte (WWx.y.i) der unterschiedlichen MRT-Sequenzen (T1, T2, KM) mit vorgegebenen Faktoren;
- eine Auswahl des Klassifikationswerts (KWi), der den höchsten Wahrscheinlichkeitswert (WWx.y.i) aller entsprechenden Klassifikationswerte (KWi) aufweist;
- eine Auswahl des Klassifikationswerts (KWi), welcjher ein höchstes Vertrauensmaß hat.

10. Verfahren nach einem der vorhergehenden Ansprüche, bei dem die Anzahl an vorgegebenen Klassifikationswerten (WWi) vier oder mehr ist.

11. Verfahren nach einem der vorhergehenden Ansprüche, bei dem die Anzahl an vorgegebenen Klassifikationswerten aus einem der folgenden Bewertungsverfahren abgeleitet sind: RAMRIS oder PsAMRIS.

12. Verfahren nach einem der vorhergehenden Ansprüche, bei dem jedes der verschiedenen Module (Mx.y) für jede der verschiedenen MRT-Sequenzen (T1, T2, KM) gesondert trainiert ist.

13. Vorrichtung zur rechnergestützten Verarbeitung digitaler Bilddaten einer Magnetresonanzeinrichtung, die dazu konfiguriert ist, Messdaten eines Untersuchungsobjekts, insbesondere einer Hand, zu erfassen und als verschiedene MRT-Sequenzen bereitzustellen, wobei die Vorrichtung einen Prozessor (TR), dass er die folgenden Schritte durchgeführt:
a) Einlesen von Messdaten eines Untersuchungsobjekts, die verschiedene MRT-Sequenzen (T1, T2, KM) umfassen;
b) Ermitteln eines ausgewählten Klassifikationswerts (AKW) aus einer Mehrzahl an vorgegebenen Klassifikationswerten (KWi, i=1..N), wobei die verschiedenen MRT-Sequenzen (T1, T2, KM) einem trainierten datengetriebenen Modell (MO) mit mehreren, den verschiedenen MRT-Sequenzen (T1, T2, KM) zugeordneten, Modulen (Mx.y) als digitale Eingangsinformation zur Verfügung gestellt werden und wobei jedes der Module (Mx.y) des trainierten datengetriebenen Modells (MO) für jeden der Mehrzahl an vorgegebenen Klassifikationswerten (KWi) einen Wahrscheinlichkeitswert (WWi) als digitale Ausgangsinformation bereitstellt, wobei der ausgewählte Klassifikationswert (AKW) derjenige Klassifikationswert der Mehrzahl an vorgegebenen Klassifikationswerten (KWi) ist, der nach einer vorgegebenen Kombination von einander aufgrund des gleichen Klassifikationswerts (KWi) zugeordneten digitalen Ausgangsinformationen jedes der Module (Mx.y) den höchsten Wahrscheinlichkeitswert (HWW) aufweist.

14. Vorrichtung nach Anspruch 13, wobei die Vorrichtung zur Durchführung eines Verfahrens nach einem der Ansprüche 2 bis 12 konfiguriert ist.

15. Computerprogrammprodukt mit Programmcode, der auf einem nicht-flüchtigen maschinenlesbaren Träger gespeichert ist zur Durchführung eines Verfahrens nach einem der Ansprüche 1 bis 12, wenn der Programmcode auf einem Computer ausgeführt wird.
